**Europäisches Patentamt**

**European Patent Office**

**Office européen des brevets**

(19)

(11) Veröffentlichungsnummer: **0 406 767 B1**

(12) **EUROPÄISCHE PATENTSCHRIFT**

(45) Veröffentlichungstag der Patentschrift: **14.09.94**

(51) Int. Cl.⁵: **C07C 67/08**, C07C 69/28, C07C 69/30, C08K 5/11

(21) Anmeldenummer: **90112599.7**

(22) Anmeldetag: **02.07.90**

(54) **Verfahren zur Herstellung eines Esters einer C22-C40-Monocarbonsäure.**

(30) Priorität: **04.07.89 DE 3921917**

(43) Veröffentlichungstag der Anmeldung:
**09.01.91 Patentblatt 91/02**

(45) Bekanntmachung des Hinweises auf die Patenterteilung:
**14.09.94 Patentblatt 94/37**

(84) Benannte Vertragsstaaten:
**AT BE CH DE ES FR GB IT LI NL SE**

(56) Entgegenhaltungen:
**EP-A- 0 310 843**
**WO-A-81/00846**
**US-A- 3 590 073**

**FIAT Final Report No. 737 (1946)**

(73) Patentinhaber: **HOECHST AKTIENGESELL-SCHAFT**

**D-65926 Frankfurt (DE)**

(72) Erfinder: **Heinrichs, Franz-Leo, Dr.**
**Hans-Fischer-Strasse 4**
**D-8906 Gersthofen (DE)**
Erfinder: **Piesold, Jan-Peter, Dr.**
**Oblatterwallstrasse 44**
**D-8900 Augsburg (DE)**
Erfinder: **Lukasch, Anton**
**Schleifweg 7**
**D-8901 Meitingen (DE)**

**Beschreibung**

Die Erfindung bezieht sich auf ein Verfahren zur Herstellung eines Esters einer $C_{22}$-$C_{40}$-Monocarbonsäure mit niedrigem Gehalt an Metallionen und geringer elektrischer Leitfähigkeit.

Technische Kunststoffe, insbesondere solche auf der Basis von Polyestern, Polycarbonaten, Polyarylethersulfonen oder Polyetherketonen zeichnen sich durch besondere mechanische und/oder thermische Belastbarkeit aus. Die in diesen Kunststoffen enthaltenen funktionellen Gruppen sind aber gegen Abbaureaktionen empfindlich. Finden Abbaureaktionen statt, so verschlechtern sich die mechanischen und thermischen Eigenschaften der Kunststoffe. Solche Abbaureaktionen, wie etwa Umesterungen, werden durch Metallionen, und hier insbesondere durch Natriumionen begünstigt.

Bei vielen Anwendungen müssen derartige Abbaureaktionen unbedingt unterdrückt werden, die Kunststoff-Formmassen dürfen deswegen nur unbedeutende Mengen an Metallionen enthalten. Gleit- und Trennmittel für derartige Formmassen müssen für sich ebenfalls den hohen Reinheitsanforderungen genügen.

Für die Herstellung von Gleitwachsen durch Veresterung von langkettigen Carbonsäuren mit Alkoholen werden nach z.B. FIAT Final Report No. 737 (1946) als Katalysatoren üblicherweise Protonkatalysatoren wie $H_2SO_4$, p-Toluolsulfonsäure, $H_3PO_2$, oder Zn- oder Sn-Verbindungen verwendet. Dabei muß der Katalysator nach der Reaktion desaktiviert werden. Die entstandenen Salze verbleiben entweder im Produkt oder müssen in einer aufwendigen Operation, beispielsweise durch Extraktion oder Destillation, abgetrennt werden.

Bekannt ist die Verwendung von Ionenaustauschern als Veresterungskatalysatoren in nichtwässrigem Medium (vgl. US 3,590,073), jedoch beschränkt sich die Anwendung auf die Herstellung niedrigmolekularer, gelöster Verbindungen.

Die Aufgabe bestand darin, ein Veresterungsverfahren zu finden, bei welchem in einem Schritt Ester langkettiger Carbonsäuren mit äußerst niedrigem Metallionenanteil erhalten werden.

Es wurde gefunden, daß die Aufgabe gelöst werden kann, wenn als Veresterungskatalysator ein saurer Ionenaustauscher eingesetzt wird.

Die Erfindung betrifft somit ein Verfahren zur Herstellung eines Esters einer $C_{22}$-$C_{40}$-Monocarbonsäure durch Veresterung der Carbonsäure mit einem Alkohol in Gegenwart eines Katalysators, dadurch gekennzeichnet, daß als Katalysator ein saurer Ionenaustauscher verwendet wird.

Für das erfindungsgemäße Verfahren wird eine $C_{22}$-$C_{40}$-,vorzugsweise $C_{24}$-$C_{34}$-Monocarbonsäure eingesetzt. Beispiele geeigneter Säuren sind Tetracosansäure, Lignocerinsäure, Cerotinsäure, Montansäure, Melissinsäure. Bevorzugt ist Montansäure, insbesondere technische Montansäure, die in Gemisch von $C_{24}$-$C_{34}$-Monocarbonsäuren darstellt und durch Oxidation von Rohmontanwachs gewonnen wird. Ganz besonders bevorzugt wird eine Säure, die eine Säurezahl größer 120, einen Na-Ionengehalt unter 10 ppm und eine Leitfähigkeit von weniger als 150 $\mu$S hat.

Geeignete Alkohole für das erfindungsgemäße Verfahren sind ein- und mehrwertige Alkohole mit 2 bis 18 C-Atomen und 1 bis 6 OH-Gruppen, vorzugsweise $C_2$-$C_6$-Polyole mit 2 bis 6 OH-Gruppen. Derartige Alkohole sind Laurylalkohol, Benzylalkohol, Ethandiol-1,2, Butandiol-1,4, Trimethylolpropan, Pentaerythrit, Glycerin. Von diesen Alkoholen werden bevorzugt Ethandiol-1,2 und Trimethylolpropan eingesetzt.

Der Veresterungskatalysator ist ein saurer Ionenaustauscher. Geeignete Ionenaustauscher sind Kationenaustauscher, welche von einer Reihe von Herstellern auf dem Markt angeboten werden. Sehr geeignet sind Poly(styrolsulfonsäuren), beispielsweise mit einer Teilchengröße von 0,1 bis 2,0 mm. Der Ionenaustauscher wird in einer Menge von 2,0 bis 0,1 Gew.-%, bezogen auf den Gesamtansatz, eingesetzt.

Die Reaktion wird in der Schmelze bei einer Temperatur von 100 bis 135, vorzugsweise 115 bis 125 °C durchgeführt. Dabei kann Atmosphärendruck oder verminderter Druck herrschen, auch Inertgasatmosphäre ist möglich. Die Reaktionszeit beträgt 1 bis 10, vorzugsweise 3 bis 7 Stunden. Der Ionenaustauscher wirkt bei der Reaktion nicht nur als Katalysator, sondern auch als Adsorptionsmittel für polare, die Thermostabilität beeinträchtigende Substanzen.

Nach der Veresterung wird der Ionenaustauscher durch Filtration entfernt und durch Waschen mit einem Lösemittel, beispielsweise Toluol oder $CH_2Cl_2$, von anhaftendem Produkt befreit. Anschließend kann er wieder eingesetzt werden. Eine Regeneration ist erst nach mehrmaligem Einsatz erforderlich.

Die nach dem erfindungsgemäßen Verfahren hergestellten Ester zeichnen sich durch eine gute Wärmestabilität, hohe Farbstabilität, sehr geringem Ionengehalt und niedrige Leitfähigkeit aus.

Die nachfolgenden Beispiele sollen die Erfindung weiter erläutern.

Zur Bestimmung der Leitfähigkeit wurden 10 g Wachs mit 100 cm³ deionisiertem Wasser versetzt und in einem 300 cm³ Erlenmeyer-Kolben 10 min unter Rückfluß erhitzt. Die wässrige Phase wurde abgetrennt und in ihr pH-Wert und Leitfähigkeit bei Raumtemperatur gemessen. Zur Messung wurde ein geeichtes Conductometer verwendet.

**Beispiel 1:**

734 g (1,76 mol) technische Montanwachssäure (SZ 134) wurde aufgeschmolzen und mit 65,2 g (1,05 mol) Ethandiol-1,2 und 4 g (0,5 Gew.-%) eines sauren Ionenaustauschers (Poly(styrolsulfonsäure, Korngröße 0,3 bis 1,2 mm) versetzt. Unter Rühren wurde der Ansatz auf 125 °C erhitzt und unter Abdestillieren des Reaktionswassers solange bei dieser Temperatur gehalten, bis eine abgetrennte Probe des Wachses eine Säurezahl von weniger als 25 hatte. Danach wurde der Ansatz filtriert.

Na 5 ppm; Leitfähigkeit 54 $\mu$S.

**Beispiel 2:**

Nach dem Verfahren des Beispieles 1 wurde technische Montanwachssäure mit Glycerin verestert. Eingesetzt wurden

917,6 g (2,2 mol) Montanwachssäure,

92 g (1,0 mol) Glycerin und

5 g (0,5 Gew.-%) des sauren Ionenaustauschers wie in Beispiel 1.

Säurezahl 23

Na 6 ppm; Leitfähigkeit 59 $\mu$S.

**Beispiel 3:**

Nach dem Verfahren des Beispiels 1 wurde technische Montanwachssäure mit Trimethylolpropan verestert. Eingesetzt wurden

917, 6 g (2,2 mol) Montanwachssäure,

134 g (1,0 mol) Trimethylolpropan und

5 g (0,5 Gew.-%) des sauren Ionenaustauschers wie in Beispiel 1.

Säurezahl < 10

Na 4 ppm; Leitfähigkeit 50 $\mu$S.

**Vergleichsbeispiel A:**

Beispiel 1 wurde wiederholt, jedoch wurden anstelle des Ionenaustauschers 0,8 g (0,1 Gew.-%) 50 %ige $H_3PO_2$ als Katalysator verwendet. Vor der Filtration des Ansatzes wurde der Katalysator mit 10 %iger NaOH neutralisiert.

Na 240 ppm; Leitfähigkeit 153 $\mu$S.

Ein Teil des Produktes wurde aufgeschmolzen und im Verhältnis 1:5 mit 5 %iger Essigsäure bei 90 °C gewaschen. Die wäßrige Phase wurde abgetrennt und der Waschvorgang wurde noch 4 mal mit deionisiertem Wasser wiederholt. Die Wachsphase wurde anschließend noch im Vakuum getrocknet.

Na 5 ppm; Leitfähigkeit 65 $\mu$S.

**Vergleichsbeispiel B:**

Beispiel 2 wurde wiederholt, jedoch wurden anstelle des Ionenaustauschers 1,0 g (0,1 Gew.-%) $H_2SO_4$ 44 %ig als Katalysator verwendet. Vor der Filtration des Ansatzes wurde der Katalysator mit 10 %iger NaOH neutralisiert.

Na 258 ppm; Leitfähigkeit 143 $\mu$S.

Waschen des Produktes, wie in Vergleichsbeispiel A beschrieben, ergab:

Na 9 ppm; Leitfähigkeit 63 $\mu$S.

**Vergleichsbeispiel C:**

Beispiel 3 wurde wiederholt, jedoch wurden anstelle des Ionenaustauschers 1,0 g (0,1 Gew.-%) 44 %ige $H_2SO_4$ als Katalysator verwendet. Vor der Filtration des Ansatzes wurde der Katalysator mit 10 %iger NaOH neutralisiert.

Na 278 ppm; Leitfähigkeit 157 $\mu$S.

Waschen des Produktes, wie in Vergleichsbeispiel A beschrieben, ergab:

Na 7 ppm; Leitfähigkeit 63 $\mu$S.

**Beispiel 4:**

Farbstabilität von PVC Rezepturen

Mit den folgenden Prüfrezepten wurden vergleichende Farbuntersuchungen mit Glycerinmontanat durchgeführt. Aus den Probemischungen wurden bei 140°C und 15/20 Upm auf einem Prüfwalzstuhl Walzfelle angefertigt. Nach je 5, 10 und 15 Minuten wurden Proben entnommen und mit einem Farbmeßgerät der Yellownessindex bestimmt.

```
          Rezept 1


          S-PVC (K 60)              100    Teile
          Zn-Stabilisator[1]          0,1    "
          Ca-Stearat                 0,3    "
          β-Diketon-Stabilisator[2]   0,3
          Epoxidiertes Sojabohnenöl   3,0    "
          MBS-Schlagzähmacher[3]      8,0    "
          Verarbeitungshilfsmittel[4] 1,0    "
          hydriertes Rizinusöl       1,0    "



     Schönungsmittel              2,0    "
     Glycerinmontanat             1,2    "



     Rezept 2


     M-PVC (K 57)               100    Teile
     Dioctylzinnglykolat          1,6    "
     MBS-Schlagzähmacher[3]       8,0    "
     Verarbeitungshilfsmittel[4]  1,2    "
     Epoxidiertes Sojabohnenöl    1,0    "
     Glycerinmonostearat          0,3    "
     Glycerinmontanat             0,6    "


     [1] Organisches Zinksalz, gelöst
     [2] Stearoyl-, Palmitoylbenzoylmethan
     [3] Vernetztes Copolymer aus Methacrylaten, Butadien und
         Styrol
     [4] Copolymer aus Methylmethacrylat und Ethylacrylat
```

Farbmessungen an Walzfellen

| | Farbe (YI) in Rezept 1 | | | Farbe (YI) in Rezept 2 | | |
|---|---|---|---|---|---|---|
| | 5 min | 10 min | 15 min | 5 min | 10 min | 15 min |
| Wachs A | -18,2 | 25,2 | 68,1 | 9,5 | 13,4 | 16,5 |
| Wachs B | -14,6 | 29 | 70 | 9,7 | 15,2 | 20,2 |
| Wachs A = Glycerinmontanat gemäß Beispiel 2 | | | | | | |
| Wachs B = Glycerinmontanat gemäß Vergleichsbeispiel B | | | | | | |

**Beispiel 5:**

Stabilität von Polyester

Ein handelsüblicher Polyester (Polyclear T 86; Fa. Hoechst) wurde mit 0,25 Teilen Ethylenglykolmontanat versetzt. Zur Bestimmung der Verarbeitungsstabilität wurden aus den Probemischungen Spritzgußplättchen mit 4 mm Stärke hergestellt und die Farbe sowie die VICAT-Erweichungspunkte bestimmt.

Zur Bestimmung der Verarbeitungsstabilität bei thermischer Dauerbelastung wurde eine Verweilzeit der Kunststoffmasse in der Spritzgußmaschine von 5, 10 und 15 Minuten eingestellt.

Für die Versuche wurde eine Spritzgußmaschine des Typs Windsor SP 50 eingesetzt, die Temperatureinstellung Einzug-Düse betrug 260, 270, 290, 300 °C, die Temperatur in der Form betrug 15 °C.

| Wachs | Farbe (YI) | | | VICAT-Erweichungspunkt | | |
|---|---|---|---|---|---|---|
| | 5 min | 10 min | 15 min | 5 min | 10 min | 15 min |
| ohne | 9,0 | 14,9 | 19,5 | 76 | 75 | 75 |
| Wachs C | 19,0 | 21,2 | 30,2 | 75,5 | 75 | 75 |
| Wachs D | 17,2 | 19,8 | 27,5 | 76 | 75 | 75 |
| Wachs C = Glycolmontanat gemäß Vergleichsbeispiel A | | | | | | |
| Wachs D = Glykolmontanat gemäß Beispiel 1 | | | | | | |

**Patentansprüche**

1. Verfahren zur Herstellung eines Esters einer $C_{22}$-$C_{40}$-Monocarbonsäure durch Veresterung der Carbonsäure mit einem Alkohol in Gegenwart eines Katalysators, dadurch gekennzeichnet, daß als Katalysator ein saurer Ionenaustauscher verwendet wird.

2. Verfahren nach Anspruch 1, dadurch gekennzeichnet, daß die $C_{22}$-$C_{40}$-Monocarbonsäure aus Montanwachsen gewonnene Montansäure ist.

3. Verfahren nach Anspruch 1, dadurch gekennzeichnet, daß die verwendete Montansäure eine Säurezahl größer 120 hat, der Gehalt an Na-Ionen unter 10 ppm liegt und die Leitfähigkeit von weniger als 150 $\mu$S beträgt.

4. Verfahren nach Anspruch 1, dadurch gekennzeichnet, daß als Alkoholkomponente ein $C_2$-$C_6$-Polyol mit 2 bis 6 OH-Gruppen eingesetzt wird.

**Claims**

1. A process for the preparation of an ester of a $C_{22}$-$C_{40}$-monocarboxylic acid by esterification of the carboxylic acid with an alcohol in the presence of a catalyst, which comprises using an acidic ion exchanger as the catalyst.

2. The process as claimed in claim 1, wherein the $C_{22}$-$C_{40}$-monocarboxylic acid is montanic acid which has been obtained from montan waxes.

3. The process as claimed in claim 1, wherein the montanic acid used has an acid number of above 120, a content of Na ions below 10 ppm and a conductivity of less than 150 $\mu$S.

4. The process as claimed in claim 1, wherein the alcohol component used is a $C_2$-$C_6$-polyol having a 2 to 6 OH groups.

**Revendications**

1. Procédé pour la préparation d'un ester d'un acide monocarboxylique en $C_{22}$-$C_{40}$ par estérification de l'acide carboxylique avec un alcool en présence d'un catalyseur, caractérisé en ce qu'on utilise en tant que catalyseur un échangeur d'ions acide.

2. Procédé selon la revendication 1, caractérisé en ce que l'acide monocarboxylique en $C_{22}$-$C_{40}$ est l'acide montanique obtenu à partir des montanwachs.

3. Procédé selon la revendication 1, caractérisé en ce que l'acide montanique utilisé a un indice d'acide supérieur à 120, la teneur en ions Na est inférieure à 10 ppm et la conductivité est inférieure à 150 $\mu$S.

4. Procédé selon la revendication 1, caractérisé en ce qu'on utilise en tant que composant de type alcoole un polyol en $C_2$-$C_6$ comportant 2 à 6 groupes OH.